# EUROPEAN PATENT APPLICATION

(11) **EP 4 516 890 A1**
(43) Date of publication of application: **05.03.2025**
(21) Application number: 23194370.5
(22) Date of filing: 30.08.2023
(51) Int. Cl.: C12M 1/00, C12M 1/36

(54) **DEVICE ASSEMBLY WITH A SIGNALLING MEANS**

(71) Applicant: Sartorius Stedim Biotech GmbH, 37079 Göttingen (DE)
(72) Inventor: GRIMM, Christian, 37079 Goettingen (DE); SCHOLZ, Jochen, 37079 Goettingen (DE); BODE, Matthias, 37079 Goettingen (DE)
(74) Representative: Prinz & Partner mbB

(57) **Abstract**

A device assembly (10) comprising a single-use device (12) for sensing or influencing a parameter of a running bioprocess. The single-use device (12) is configured to have at least two different defined functional states, a first functional state being an inactive delivery state in which the single-use device (12) is sterile and inoperable according to its intended use, and a second functional state being an active use state in which the single-use device (12) is operable according to its intended use. The device assembly (10) further comprises a signalling means (34) for indicating a current functional state of the single-use device (12) to a user. Each functional state is associated with a distinct signal (38, 40, 42). The device assembly (10) is configured such that a transfer from the first functional state to any other functional state of the single-use device (12) is irreversible and/or permanently prevents the signalling means (34) from indicating the signal (38) associated with the first functional state.

## Description

The invention relates to a device assembly comprising a single-use device for sensing or influencing a parameter of a running bioprocess and a signalling means for indicating a current functional state of the single-use device to a user.

There is a clear trend in the biopharmaceutical industry to use more single-use equipment in clinical trial manufacturing or even commercial manufacturing, both following so-called "current good manufacturing practice" regulations (cGMP). Especially in single-use cell culture and transfer processes, single-use sensors and actuators become more and more common. Thereby, single-use sensors are used to control critical process parameters such as the pH value or the capacity of a sample, and single-use actuators are used for controlling devices such as pumps, valves or sample loading devices. Unlike fixed or permanent sensors, which remain in the pharmaceutical device after installation and commissioning, single-use devices have to be replaced after each usage by a new sterile single-use device to avoid contaminations.

Typically, single-use devices such as single-use sensors have multiple functional states such as a sterile delivery state, a measuring state or a calibration state. For proper use of this kind of sensor or actuator it is important for a user to know in which of the functional states the single-use device currently is to avoid for example reusing a single-use sensor which should not be used anymore. It is also important for the user to know when and how the single-use device can securely be transferred from one functional state to another.

Various single-use sensors are known from the prior art having different functional states to perform different functions at different points in time. However, it is often not possible for a user to unambiguously identify in which current functional state the single-use sensor is. Typically, no distinction is made for example between an unused sterile single-use sensor and a used unsterile single-use sensor, which might lead to an unintended use of an unsterile single-use sensor and thus in worst case to a contamination of the sample. To avoid this, the user of the single-use device has to log the current functional state by hand, which in turn is time-consuming and prone to errors.

WO 2013/026512 A1 discloses an augmented reality system for industrial manufacturing and laboratory use in the field of pharmaceutics and biopharmaceutics. The disclosed augmented system can detect the location and/or status of a marker representative of a particular assembly component to decide whether the connection between two assembly components is correctly established. However, it is not possible to distinguish between individual functional states of the sensor or actuator.

The object of the invention is therefore to provide a single-use device that has at least two functional states and at least two corresponding unique signals representative for the current functional state and thereby enable the recognition of the current functional states of the single-use device for a user. Additionally, the device should be able to support the user when changing the current functional state to the preferred next functional state and to enable the documentation of the current functional state of the single-use device.

The above mentioned problem is solved by a device assembly according to claim 1. The device assembly according to the invention comprises a single-use device for sensing or influencing a parameter of a running bioprocess.

The single-use device is configured to have at least two different defined functional states. A first functional state is an inactive delivery state in which the single-use device is sterile and inoperable according to its intended use. The single-use device is preferably delivered in that first functional state. A second functional state represents an active use state in which the single-use device is operable according to its intended use. The device assembly further comprises a signalling means for indicating a current functional state of the single-use device to a user. Each functional state is associated with a distinct signal. This means that the signal for the first functional state differs from the signal for the second functional state. The device assembly is configured such that a transfer from the first functional state to any other functional state of the single-use device is preferably irreversible. In case the transfer is not irreversible, at least the signalling means is permanently prevented from indicating the signal associated with the first functional state symbolizing an unused sterile single-use device, i. e. the single-use device cannot be indicated as "unused" anymore.

The invention is generally based on the finding that the user receives clearly defined unambiguous feedback about the current functional state of the single-use device by assigning a distinct signal to each functional state. In particular, the user is able to distinguish between a sterile unused single-use device and a single-use device which has already been used, because in the sterile state of the single-use device the signalling means issues a first signal, while in the used state of the single-use device the signalling means issues a second signal that is clearly different from the first signal.

In the following, the scope of several terms used in connection with the invention will be explained:
With respect to the term "single-use", it should be noted that the skilled person can precisely distinguish between dedicated single-use components (disposables) on the one hand, which are not intended for cleaning after their one-time use but for a disposal, and reusable components, which are intended for repeated use, on the other hand. In each case, it can be clearly determined whether a component for a device assembly is disposable or reusable based on its material and design. The components are clearly distinguished from one another in the specialist circles with regard to their intended use (single-use versus reusable), and the respective advantages and disadvantages are subject of detailed discussions in the art. Moreover, laboratory equipment suppliers specifically offer and market single-use components with clear labelling.

The single-use device which is delivered in the first functional state is inoperable according to its intended use, meaning, that the single-use device cannot be used for measuring or controlling or any other functional usage in this state. To perform anything such as measuring or controlling something with the single-use device, the state of the single-use device first has to be transferred from the first functional state to another functional state.

Generally, a state of the single-use device can be switched from one functional state to any other functional state. When switching the functional state, also the associated distinct signal of the functional state of the single-use device changes. However, the transfer from the first functional state (inactive delivery state) to another functional state should effectively be irreversible, so that the user cannot transfer the single-use device from any other functional state back to the first functional state. In other words, the user cannot accidentally mark a device that has already been used with a signal which is representative for an unused single-use device. The irreversibility of the transfer back to the first functional state can be technically realised, for example, by a latching mechanism which mechanically prevents the transfer back to the first functional state. It is also possible to prevent the transfer to the first functional state by an automatic control.

Nevertheless, it might be possible for some single-use devices to prepare them for reuse by autoclaving. In such a case, the device assembly can be transferred back from any other functional state to the first functional state, but the new signal for the current functional state, which is technically the first functional state, differs from the original signal for the first functional state indicating a single-use device which is sterile and not yet operable. This means, if a single-use device is transferred from any other functional state back to the first functional state, the signal after the transfer will not be the same signal as for a single-use device in the inactive delivery state, so the signalling means does not indicate the signal associated with the first functional state again. Thus, the indication of the first functional state cannot be reversibly changed. In this way, the security regarding the usage of a sterile single-use device can be significantly increased.

The term "indication of a current functional state" is understood to encompass different types of indication, such as a visual indication, an acoustic indication or a haptic feedback. A combination of several types of indication is also possible. In addition, the signalling means may be a display or feedback directly on the disposable device, for example at a display that is part of the disposable device, or the single-use device may trigger an output on a remote device such as a remote display or computer.

The single-use device for sensing or influencing a parameter of a running bioprocess can be any type of sensor for detecting or measuring one or more physical or chemical properties. Preferably, the device has more than two different functional states to which it can be transferred mechanically, either manually or automatically.

Possible sensors include a pH-sensor, preferably an electrochemical pH-sensor, a conductivity sensor, a capacitance sensor, an impedance sensor, a temperature sensor, a sensor for concentration measurement, and an optochemical sensor. In connection with the present invention, the term "single-use sensor" especially refers to a portion, part or component of the sensor that comes into direct contact with a liquid or gaseous sample, e. g. flowing through a tube line or being integrated into a single-use container, such as bioreactor, via a port means or the like. In principle, the other parts of the sensor can be reusable.

In another aspect of the invention, the device assembly comprises a single-use device which is one of the following actuators: a pump or a pump head, a valve, a sampling device, preferably an automatic sampling device. Again, only the portion, part or component of the actuator that comes into direct contact with the sample needs to be configured for a single-use.

Preferably, the single-use device is configured to have a defined third functional state in which it is prepared for calibration, reference check, adjustment such as tare or zeroing, intermediate storage or passivation and cleaning. In the third functional state the single-use device is already operable and unsterile similar to the second functional state. However, the single-use device is not measuring or detecting or influencing a parameter of a running bioprocess in the third functional state. Rather, the single-use device is in a functional state where it is not sterile anymore but not performing its real function in a process. Generally, since the single-use device is designed to be always in one defined functional state, a single-use device which is neither sterile nor measuring can be transferred into the third functional state and have a signalling means indicating the third functional state. Thus, the third functional state can serve as an intermediate state between a sterile state and a state in which the device is performing its intended use.

Preferably, the single-use device is configured such that a transfer from the second functional state to the third functional state or from the third functional state to the second functional state is reversible and causes a signalling means to indicate the signal associated with the second or third functional state, respectively. This enables for example a recalibration during a measuring process or to perform a post-use calibration, which is typically needed under cGMP condition. In parallel to the transfer of the single-use device from one functional state to another functional state, the signalling means also changes its signal, so that the user is immediately informed in which functional state the single-use device currently is or to which functional state the single-use device has been transferred.

According to another aspect of the invention, the single-use device is configured to have a defined fourth functional state in which the single-use device, after its intended use, is made inoperable. A single-use device transferred to the fourth functional state shall not be used anymore. The deactivation of the single-use device can be realised, for example, also by a latching mechanism or an automatic control which prevents the single-use device to be transferred from the fourth functional state to any other functional state. The fourth functional state might become important for types of single-use devices which were designed to be used just for a specific time period or a fixed number of recalibration steps. Preferably, the single-use device is transferred to the fourth functional state after a certain period of use or a certain number of calibration and/or cleaning cycles, which should not be exceeded.

However, both the third functional state and the fourth functional states are optional functional states. Thus, a single-use device having only a fist, a second and a fourth functional state as well as a single-use device having only a first, a second and a third functional state are also to be considered as embodiments of the invention.

The single-use device can be configured such that a transfer from any of the other functional states to the fourth functional state is irreversible and/or the signalling means is permanently prevented from indicating a signal associated to the other functional states. Preferably, the transfer to the fourth functional state is irreversible, so that it is not possible for a user to switch from the fourth functional state back to any other functional state by accident. This "out-of-use mode" provides a higher process reliability and is especially important while working under GMP conditions. However, similar to the irreversibility of the first functional state, it might also be possible to mechanically transfer the single-use device from the fourth functional state to any other functional state, but then the signal of the signalling means is prevented from issuing the signal of the other functional state into which the single-use device has been transferred to. In other words, in case the single-use device is transferred from the fourth functional state to any other functional state, the signalling means will not output the signal of the current functional state but the signal of the fourth functional state.

According to aspect preferred embodiment of the invention, the signal of the signalling means includes distinct indication elements, and each functional state of the single-use device is associated with the different indication element. The different indication elements guide the user through the different functional states and indicate the current functional state. When changing the functional state, the indication element changes as well, if the change is allowed. The indication element can either be a passive element or an active element. Passive elements are elements which are visible whereas active elements are all kind of elements, which send an active signal to the user who perceives it through other senses, such as hearing and feeling.

Passive indication elements can include different symbols and/or text, preferably in different colours. Thus, each functional state of the single-use device is associated to a distinct signal comprising at least one distinct indication element such as a distinct text or word for each functional state or a different symbol. In principle, it is possible to also have the same type of symbol for each functional state but in a different colour, wherein each colour is distinct for a functional state. The different indication elements having different colours can be laser markings or other types of markings, whereby the indication element only become visible when the single-use device is set to the corresponding functional state. For example, in case the single-use device is set to the first functional state, the indication element signals the signal for the first functional state whereas the indication elements for the other functional states are covered.

The indication element that is associated to a current functional state of the single-use device can be made visible by illumination and/or on a display. The display can either be located on the single-use device or remote from the single-use device. The illumination can either be in one colour or multi-coloured by using multi-coloured lamps. It is also possible to have an illuminating indication element comprising different coloured regions or symbols for example, labelled by laser marking. Such illuminating indication elements can either be realised by connecting a fibre-optic cable to the indication element or by an integrated lamp which is provided with energy either by an external energy source or by an internal energy source.

In case the indication element is an active indication element, it can include a motion like a distinct vibration pattern and/or sound and/or haptic feedback.

Of course, it is possible, to combine a passive indication element such as a text together with an active indication element such as a distinct motion or vibration of the single-use device.

The single-use device can be configured such that a transfer from one functional state to another functional state can be performed manually, thereby automatically revealing or triggering output of the indication element associated to the current functional state. In other words, whenever the single-use device is transferred to another functional state either manually or automatically, the signal of the signalling means changes automatically, if the transfer is allowed, without interaction of the user. This leads to the advantage that the user does not have to take care of indicating or documenting the functional state of the single-use device even if the user switched the single-use device from any functional state to another functional state manually. This makes the whole single-use device more user-friendly and protects at the same time against a change of one functional state to another happening unnoticed, e. g. when the user has forgotten to note the change.

According to another aspect of the invention, the device assembly comprises a control unit connected to the single-use device.

The control unit can be configured to prompt a user to perform or automatically indicate a transfer of the single-use device from one functional state to another functional state. The control unit in principle can be any electric device which is configured to prompt a user or initiate the transfer from one functional state to another functional state. The usage of such a control unit simplifies the working routine of the user and increases the process reliability. However, again the transfer from the fourth functional state to any other functional states as well as the transfer back to the first functional state are impossible or will not be indicated as such.

According to one variant, the signalling means is part of or connected to the control unit. In case the signalling means is part of the control unit, the control unit is preferably a display and the signalling means comprises preferably passive indication elements. However, it is also possible that the signalling means is not part of the control unit itself but connected to it by a line ore wirelessly. In such a case, the signalling means can be part of the single-use device which is connected to the control unit to be automatically controlled and/or monitored by the control unit. To transfer the information of the signalling means to the control unit, the control unit and thus the signalling means are connected by a line or wirelessly.

Optionally, the control unit includes or is further connected to a storage unit which is configured to save the current functional state of the single-use device. This enables the documentation of the current functional state as well as the transfer from one functional state to another functional state. Also the interaction between the user and the device assembly can be recorded and documented.

The control unit can either be attached to the single-use device or it can be located remote from the single-use device. Depending on the size of the control unit, it can be attached to the single-use device such as a pump. In case the single-use device is smaller or the control unit is larger, it is preferred to have a control unit located remote from the single-use device and to connect the control unit to the single-use device by a line or wirelessly.

Further features and advantages of the invention will become apparent from the following description and from the accompanying drawings to which reference is made. In the drawings:
Figure 1 shows a device assembly according to the invention with a pH-sensor as a single-use device in a first functional state;
Figure 2 shows the device assembly according to Figure 1 with a single-use device in a second functional state;
Figure 3 shows the device assembly according to Figure 1 with a single-use device in a third functional state;
Figure 4 shows the device assembly according to Figure 1 with a single-use device in the fourth functional state;
Figure 5 shows a single-use device according to the invention with a passive indication element comprising a multi-coloured symbol;
Figure 6 shows a single-use device according to the invention with a passive indication element, wherein the indication element is made visible by coloured illumination;
Figure 7 shows a single-use device according to the invention with an indication element comprising a multi-coloured LED; and
Figure 8 shows a single-use device according to the invention with an indicator.

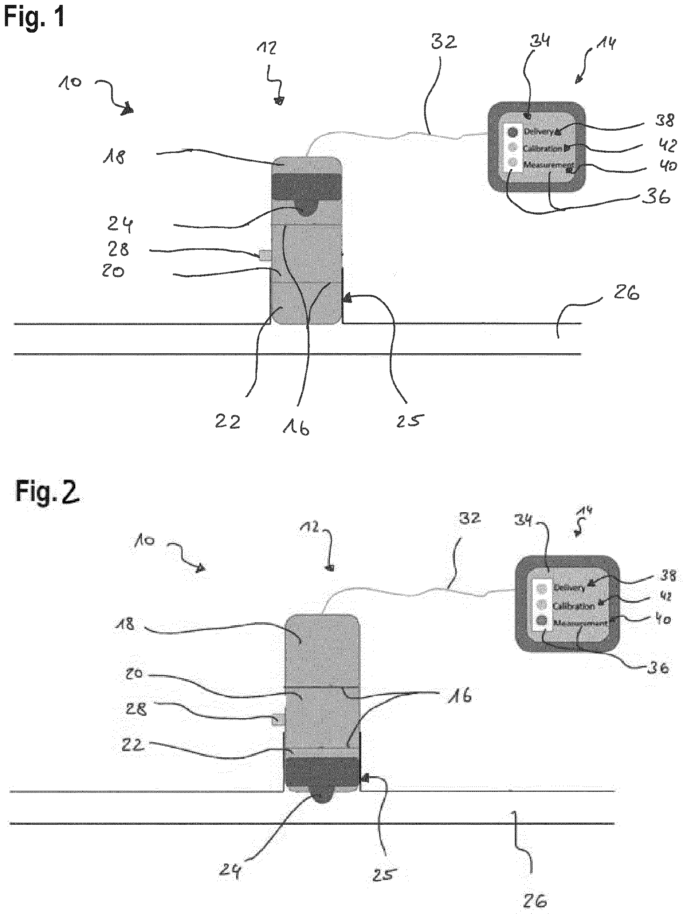

Figures 1 to 4 show a device assembly 10 comprising a single-use device 12, here a single-use pH sensor for a bioreactor, and a control unit 14 located remote from the single-use device 12, which is described later in detail.

The single-use device 12 has three or four different defined functional states, to which it can be transferred either manually or automatically.

A first functional state, as shown in Figure 1, corresponds to an inactive delivery state in which the single-use device 12 is sterile and inoperable according to its intended use. Thus, the single-use device 12 cannot be used for measuring the pH value of a sample yet.

A second functional state, as shown in Figure 2, corresponds to the state in which the single-use device 12 is set while measuring according to its intended use. In this state, the single-use device 12 is not sterile anymore as it is currently in use.

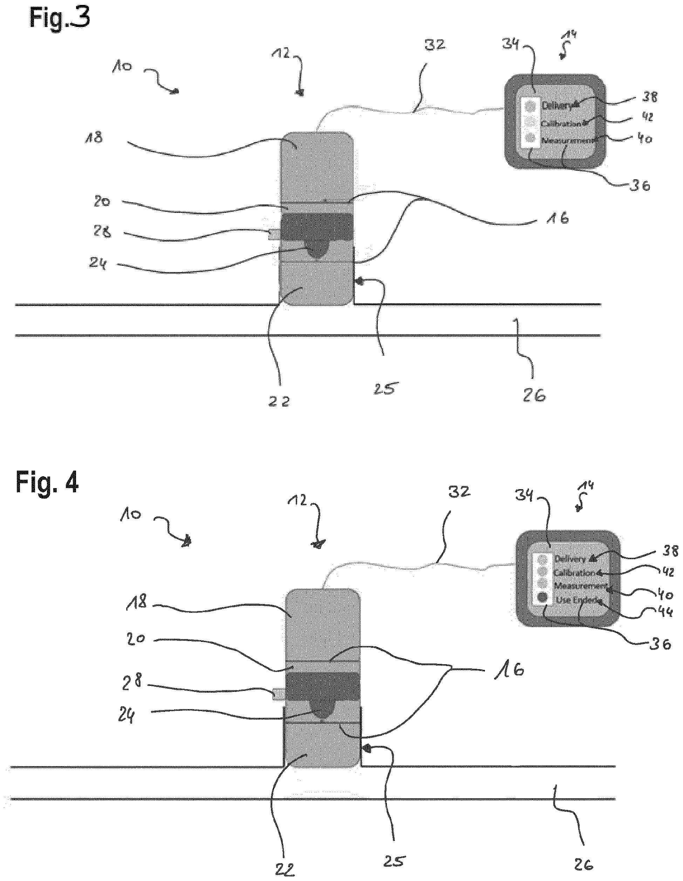

The single-use device 12 also has a third functional state, as shown in Figure 3, to which it is transferred for a calibration, a reference check, an adjustment like tare or zeroing, an intermediate storage or passivation and/or cleaning of the single-use device 12.

Optionally, the single-use device 12 can also be transferred into a fourth functional state, as shown in Figure 4, indicating a single-use device 12 which should no longer be used.

In order to technically implement these three or four defined functional states, the inside of the single-use device 12 is separated by two compartment separations 16 to generate three different compartments 18, 20, 22. These compartment separations 16 are fixedly mounted in the inside of the single-use device 12. The distance between the single compartment separations 16 can be freely chosen so that the size of each compartment can be freely determined according to the preferred volume.

An upper compartment 18 is located in the upper part of the single-use device 12, a middle compartment 20 is located in the middle part of the single-use device 12 and a lower compartment 22 is located in the lower part of the single-use device 12.

Inside the single-use device 12, a probe tip 24 is movably mounted so that it can be moved from one compartment to another compartment. Depending on the functional state of the single-use device 12, the probe tip is either located in the upper compartment 18, the middle compartment 20 or the lower compartment 22.

When the single-use device 12 is delivered, it is set to the first functional state in which the probe tip 24 is located in the upper compartment 18. In this state, the single-use device 12 is sterile, unused and inoperable.

In order to prepare the single-use device 12 for measuring or calibrating, the single-use device 12 is unpacked and the probe tip 24 is moved either manually or automatically into the middle compartment 20 for calibrating the probe tip 24, or into the lower compartment 22 for measuring a sample.

Once the probe tip 24 is moved from the upper compartment 18 to any other compartments 20 or 22, the probe tip 24 cannot be moved back to the upper compartment 18 again. Thus, whenever the single-use device 12 is switched from the first functional state to any other functional state, it is impossible to switch the single-use device 12 back to the first functional state. Since the transfer of the probe tip 24 from the upper compartment 18 to any other compartment 20, 22 is irreversible also, the transfer from the first functional state to any other functional state is irreversible.

This irreversibility of the transfer is realized by a latching mechanism which physically prevents the probe tip 24 to move back to the upper compartment 18. When the single-use device 12 is controlled automatically, a movement of the probe tip 24 into the upper compartment 18can also be prohibited by a control algorithm.

However, some single-use devices do not have such a latching mechanism or the like, so the probe tip 24 can physically be moved from any other compartment 20 or 22 back to the upper compartment 18. This case will be discussed further below.

When the single-use device 12 is transferred to the second functional state, it is operable according to its intended use, such as measuring the pH value of a sample. For performing its intended use, the single-use device 12 is mounted to a sensor port 25 of a tube line 26, preferably a single use tube line 26. The probe tip 24 is moved from the upper compartment 18 or the middle compartment 20 to the lower compartment 22 so that it gets in contact with the sample in the tube line 26 to be measured.

In case the single-use device 12 needs to be calibrated, the probe tip 24 is moved from the upper compartment 18 or the lower compartment 22 to the middle compartment 20 and thereby transferred from the first or the second functional state to the third functional state.

The calibration can be performed multiple times, either in a row or with some measuring periods in between. This ensures that the single-use device 12 can be calibrated before, during and/or after measuring.

During a calibration step, a calibration solution is inserted through an inlet 28 into the middle compartment 20 so that at least parts of the middle compartment are filled with the calibration solution. When the probe tip 24 is surrounded by the calibration solution, the calibration step can be executed.

After calibrating the single-use device 12, it can either be removed from the sensor port 25 of the tube line 26 and transferred into the fourth functional state (e.g. if calibration has failed), or it can be transferred into the second functional state and start/continue measuring the sample inside the tube line 26.

Of course, the probe tip 24 can also be transferred directly from the upper compartment 18 via the middle compartment 20 to the lower compartment 22 without resting at the middle compartment 20.

After several calibration/measuring cycles, after a certain time of use or when the measuring or calibration is finished, the single-use device 12 can be transferred into the fourth functional state indicating that the single-use device 12 should no longer be used. In this functional state, the single-use device 12 is inoperable again. This fourth functional state is especially useful for single-use sensors with a maximum number of calibration/measuring cycles or a limited service life, or simply to ensure that the single-use device 12 will not be used anymore after the bioprocess has been terminated..

As shown in the Figures 1 to 4, the device assembly 10 comprises a control unit 14 which is located remote from the single-use device 12. However, the control unit 14 can also be attached to the single-use device 12, preferably to a large single-use device 12.

The main function of the control unit 14 is to specify and visualize the current functional state of the single-use device 12. Additionally, the control unit 14 can be configured to control the position and the movement of the probe tip 24 inside the single-use device 12 so that the control unit 14 can control the transfer the single-use device 12 from one functional state to another.

To this end, the single-use device 12 has to be connected to the control unit 14 by an electric line 32. Via this electric line 32, the control unit 14 can send information to the single-use device 12 about when to change the current functional state, and/or the single-use device 12 can send information regarding its current functional state. This information can be indicated to the user by the control unit 14.

To display the current functional state of the single-use device 12, the control unit 14 comprises a signalling means 34 sending signals to a user. The signalling means 34 comprises different indication elements 36, wherein each indication element 36 is associated with a different functional state. As shown in Figures 1 to 4, the signal associated with one functional state includes two different indication elements, namely a coloured symbol and a text. This makes it easier for the user to perceive the current functional state of the single-use device 12.

Generally, the indication element 36 can be any type of signal such as a symbol, a text, a light, a motion, a sound or a haptic signal, as long as each signal is distinct for a defined functional state. The indication element can be made visible by illumination and/or on a display, depending on whether the control unit 14 includes a display or not.

Figure 1 shows the device assembly 10 with a single-use device 12 in the first functional state. Therefore, the probe tip 24 is in the upper compartment 18 and the signalling means 34 of the control unit 14 displays a first signal 38 with distinct indication elements 36 for the first functional state. As indicated by the two compartment separations in this functional state, the probe tip 24 can be moved from the upper compartment 18 either to the middle compartment 20 or to the lower compartment 22 depending on the desired function to be carried out next. However, preferably it is not possible, to transfer the single-use device 12 from any other functional state back to the first functional state, meaning the transfer from the first functional state to any other functional state of the single-use device 12 is irreversible.

However, some single-use devices 12 can physically be transferred back into the first functional state. In order to prevent reuse of the single-use device 12, the signal at the signalling means 34 is not switched to the first signal 38.

Figure 2 shows the device assembly 10 after being prepared for measuring or while it is measuring. The probe tip 24 is moved into the lower compartment 22 to get in contact with the sample inside the single-use tube line 26. Of course, the single-use tube line 26 could also be any container, such as single-use bag of a bioreactor, for example. The signalling means 34 of the control unit 14 shows a second signal 40 indicating that the device assembly 10 is in the second functional state. The second signal 40 is distinct for this functional state.

The device assembly 10 can be transferred from the first functional state or the second functional state to the third functional state which is shown in Figure 3. In the third functional state, the single-use device 12 is prepared for calibration, currently calibrating, performing a reference check, under adjustment such as tare or zeroing, intermediately stored, passivated or currently cleaned. During this functional state, the probe tip 24 is located in the middle compartment 20 to get into contact with the calibration solution or another suitable solution inserted into the middle compartment 20 through the inlet 28. The control unit 14 displays a third signal 42 which is distinct for the third functional state and includes two different indication elements 36 here. Starting from this functional state, the single-use device 12 can be transferred either into the second functional state or into the fourth functional state.

Figure 4 shows the device assembly 10 in the fourth functional state, indicating that the single-use device 12 should no longer be used. In this optional fourth functional state the probe tip 24 is transferred into the middle compartment 20 or the upper compartment 18 and is fixed permanently and preferably irreversible to make the single-use device 12 inoperable. The control unit 14 displays a fourth signal 44 which is distinct for the fourth functional state.

In case the single-use device 12 is transferred from one functional state into the fourth functional state manually, fixation can be realized by a latching mechanism similar to the latching mechanism preventing the single-use device 12 from being transferred back to the first functional state. In case the single-use device 12 is transferred automatically by the control unit 14, a control algorithm prevents the control unit 14 from transferring the single-use device 12 from the fourth functional state to any other functional state.

Even if it is possible to physically transfer the single-use device 12 from the fourth functional state back to any other functional state again, the signal of the signalling means would still display the fourth signal 44. Accordingly, it is still signalized to the user, that the single-use device 12 should no longer be used.

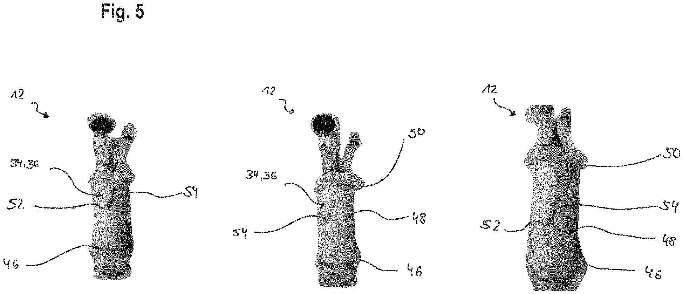

Figures 5 to 8 show different single-use devices 12 with a signalling means 34 comprising different types of indication elements 36 signalling the distinct current functional state of the single-use device 12.

The single-use device 12 has a cylindrical housing 46 comprising two parts. An outer part 48 of the housing 46 encloses an inner part 50 of the housing 46. The outer part 48 can be twisted or moved relative to the inner part 50 of the housing 46. The twisting or moving of the outer part 48 relative to the inner part 50 can be stopped at three defined positions, wherein the first position is representative for the first functional state of the single-use device 12, the second position is representative for the second functional state and the third position is representative for the third functional state.

The outer part 48 has at least one opening 52 and a portion of the inner part 50 placed underneath the opening 52 comprises at least one indication element 36 such as different coloured symbols printed onto the surface of the inner part 50. Thus, the openings 52 of the outer part 48 and the symbols of the inner part 50 together form the signalling means 34. Accordingly, here the signalling means 34 is part of the single-use device 12.

By turning or twisting the outer part 48 relative to the inner part 50, the functional state of the single-use device 12 can be changed and simultaneously the indication element 36, which becomes visible through the openings 52, is changed as well according to the current functional state. In other words, when the outer part 48 is turned from the first functional state to the second functional state by twisting the outer part 48 relative to the inner part 50, the position of the opening 52 changes and another indication elements 36 becomes visible, namely the second signal 40 indicating the second functional state.

As shown in Figure 5, the indication element 36 includes passive symbols such as differently coloured strips 54, wherein each colour of each indication element 36 corresponds to a defined functional state of the single-use device 12. The different coloured strips are laser markings, although the indication elements 36 can also be printed text, such as words like "storage", "calibration" and "application" indicating the defined functional state of the single-use device 12 as it is shown in Figures 1 to 4.

The single-use device 12 of Figure 5 on the left hand side is set to the first functional state. The signalling means 34 comprises the indication element 36 with the first signal 38. The single-use device 12 in the middle is set to the third functional state symbolized by an indication element 36 visualizing the third signal 42. The second signal 40 is visible on the single-use device 12 on the right hand side of Figure 5.

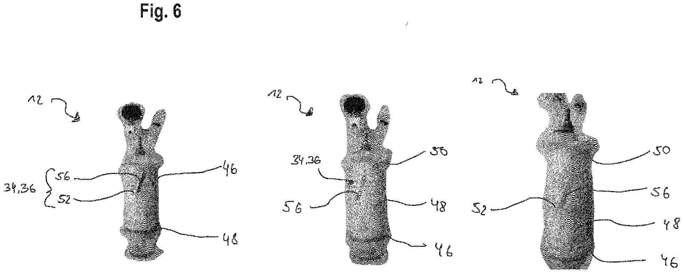

As shown in Figure 6, the symbols of the indication elements 36 are passive, illuminated symbols 56 which become visible by illumination. The illuminated symbols 56 are placed on the inner part 50 of the single-use device 12 and are released when the openings 52 of the outer part 48 are on top of the illuminated symbols 56. Thus, when twisting the outer part 48 of the housing 46 relative to the inner part 50, different illuminated symbols, preferably in different colours, become visible and indicate the current functional state of the single-use device 12.

If the indication element 36 associated to the current functional state of the single-use device 12 comprises symbols designed as an illuminated symbol 56, the symbols can either be illuminated via a connected fibre optic cable or an integrated light source connected to either an internal or external energy source. Each illuminated symbol 56 can have its own energy source or multiple illuminated symbols 56 can be provided with energy by one energy source.

The single-use device 12 shown in Figure 6 on the left hand side is set to the first functional state, so the indication element 36 visualizes the symbol representative for the first functional state. The single-use device 12 in the middle is currently in the third functional state and the indication element 36 associated with the third functional state is visible. This indication element 36 is also an illuminated symbol 56, but it has a different colour than the illuminated symbol 56 of the first signal 38. The single-use device 12 shown on the right hand side in Figure 6 shows a third illuminated symbol 56 which is representative for the second signal 40.

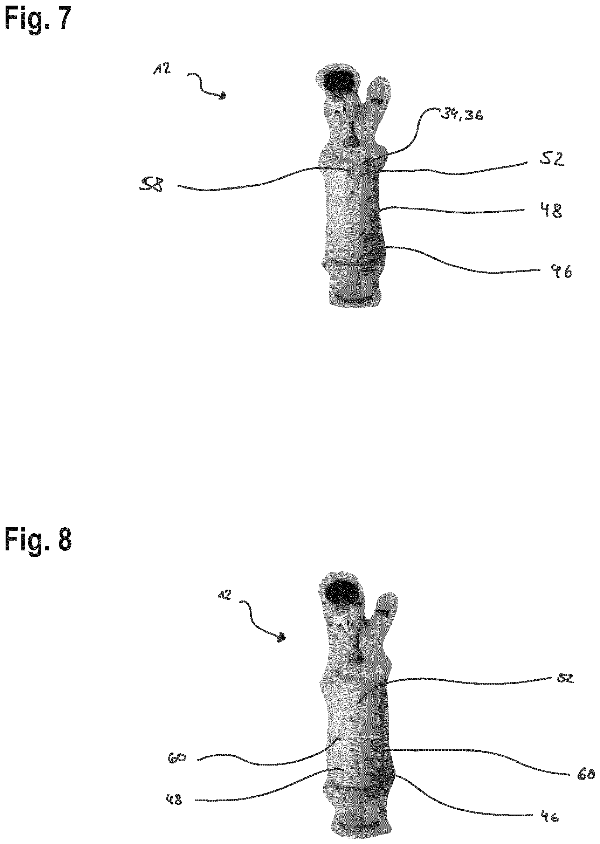

Figure 7 shows a single-use device 12 with a third variant of an indication element 36. The signalling means 34 here comprises a display element 58 which represents a signal of the indication element 36. The display element 58 comprises preferably at least one LED lamp or a display with many LED lamps, which both are part of the single-use device 12, more particular of the inner part 50 of the housing 46 of the single-use device 12.

The single-use device 12 also has different functional states. To transfer the single-use device 12 from one state to another functional state, the outer part 48 is manually twisted relative to the inner part 50 of the housing 46 of the single-use device 12.

In contrast to the indication elements 36 shown in Figure 5 and 6, the housing 46 of the single-use device 12 further comprises a switch (not shown), preferably a capacitive, inductive, electrically conductive or tactile switch, having different switch positions. When twisting the outer part 48 relative to the inner part 50 of the housing, the position of the switch is changing. The switch has as many switching positions as the single-use device 12 has functional states.

Each time the functional state of the single-use device 12 is switched, the switching position of the switch changes accordingly, causing the indication element 36 to change from a display element 58 associated to one functional state to a display element 58 associated to the new functional state.

The display elements 58 are provided with energy either by an internal energy source or an external energy source, which are not shown here. Of course, the single-use device 12 can comprise more than one display element.

Figure 8 shows a single-use device 12 similar to the one shown in Figure 7. To change the functional state of the single-use device 12, the outer part 48 is moved or twisted relative to the inner part 50, whereby the switch position of the switch is changing accordingly.

In contrast to Figure 7, the transfer from one functional state to another functional state can be performed automatically, initiated by the control unit 14 (not shown here). Therefore, also the signalling means 34 with the indication element 36 is not placed on the single-use device 12 but remote on the control unit 14 which is connected to the single-use device 12.

The control unit 14 visualizes the current functional state of the single-use device 12 either analogously or digitally.

The control unit 14 is further connected to or comprises a memory which is configured to store the current functional state of the single-use device 12. Therefore, the current functional state as well as the transfers from one functional state to another functional state can be documented easier; preferably the documentation can be done in a digital manner. This improves the intended use of the single-use sensor 12 and its documentation according to the GMP conditions.

Furthermore, the control unit 14 together with the memory can activate indicators 60 placed on the single-use device 12. These indicators 60 are symbols, preferably illuminated arrows, indicating the user to now change the functional state of the single-use device 12. The indicators 60 also indicate in which direction the outer part 48 of the house 46 should be twisted. These indicators 60 therefore assist the user with transferring the single-use device 12 into another functional state.

This is helpful, for example, when the single-use device 12 is in the third functional state and the calibration has just finished. By illuminating an arrow, signalling the turning direction, the user is prompted to transfer the single-use device 12 from the third functional state to the second functional state and to start the measurement.

The single-use device 12 can further comprise a fourth functional state to which it can be transferred either manually or automatically by the control unit 14. This functional state can also be visualized by one of the aforementioned symbols on the control unit 14 or on the single-use device 12.

All these different symbols of the signalling means 34 help the user to identify and visualize the current functional state of the single-use device and prevent him from using a single-use device which should no longer be used. Additionally, the user is assisted with transferring the single-use device from one functional state to another functional state.

It is to be noted that the features of the above-described embodiments can be combined in other suitable ways.

## Claims

1. A device assembly (10) comprising
a single-use device (12) for sensing or influencing a parameter of a running bioprocess, the single-use device (12) being configured to have at least two different defined functional states, a first functional state being an inactive delivery state in which the single-use device (12) is sterile and inoperable according to its intended use, and a second functional state being an active use state in which the single-use device (12) is operable according to its intended use; and
a signalling means (34) for indicating a current functional state of the single-use device (12) to a user;
each functional state being associated with a distinct signal (38, 40, 42),
the device assembly (10) being configured such that a transfer from the first functional state to any other functional state of the single-use device (12) is irreversible and/or permanently prevents the signalling means (34) from indicating the signal (38) associated with the first functional state.

2. The device assembly (10) according to claim 1, **characterized in that** the single-use device (12) is one of the following sensors: a pH-sensor, preferably an electrochemical pH-sensor; a conductivity sensor; a capacitance sensor; an impedance sensor; a temperature sensor; a sensor for concentration measurement; an optochemical sensor.

3. The device assembly (10) according to claim 1, **characterized in that** the single-use device (12) is one of the following actuators: a pump or a pump head; a valve; a sampling device, preferably an automatic sampling device.

4. The device assembly (10) according to one of the preceding claims, **characterized in that** the single-use device (12) is configured to have a defined third functional state in which the single-use device (12) is prepared for a calibration, a reference check, an adjustment, an intermediate storage or a passivation and cleaning.

5. The device assembly (10) according to one of the preceding claims, **characterized in that** the single-use device (12) is configured such that a transfer from the second functional state to the third functional state or from the third functional states to the second functional state is reversible and causes the signalling means (34) to indicate the signal (40, 42) associated with the second or third functional state, respectively.

6. The device assembly (10) according to one of the preceding claims, **characterized in that** the single-use device (12) is configured to have a defined fourth functional state in which the single-use device (12), after its intended use, is made inoperable and shall not be used anymore.

7. The device assembly (10) according to claim 6, **characterized in that** the single-use device (12) is configured such that a transfer from any of the other functional states to the fourth functional state is irreversible and/or permanently prevents the signalling means (34) from indicating a signal (38, 40, 42) associated to the other functional states.

8. The device assembly (10) according to one of the preceding claims, **characterized in that** the signals (38, 40, 42) of the signalling means (34) include distinct indication elements (36), each functional state of the single use device (12) being associated with a different indication element (36).

9. The device assembly (10) according to claim 8, **characterized in that** the indication elements (36) include different symbols and/or text, preferably in different colours.

10. The device assembly (10) according to claim 9, **characterized in that** the indication element (36) associated to the current functional state of the single-use device (12) is made visible by illumination and/or on a display.

11. The device assembly (10) according to claim 8, **characterized in that** the indication elements (36) include different motions and/or sounds and/or haptic feedbacks.

12. The device assembly (10) according to one of the preceding claims, **characterized in that** the single-use device (12) is configured such that a transfer of the single-use device (12) from one functional state to another functional state can be performed manually, thereby automatically revealing or triggering output of the indication element (36) associated to the current functional state.

13. The device assembly (10) according to one of the preceding claims, **characterized in that** the device assembly (10) comprises a control unit (14) connected to the single-use device (12).

14. The device assembly (10) according to one of the preceding claims, **characterized in that** the control unit (14) is configured to prompt a user to perform or to automatically initiate a transfer of the single-use device (12) from one functional state to another functional state.

15. The device assembly (10) according to one of the preceding claims, **characterized in that** the signalling means (34) is part of or connected to the control unit (14).

16. The device assembly (10) according to one of claims 13 to 15, **characterized in that** the control unit (14) includes or is connected to a storage unit which is configured to save the current functional state of the single-use device (12).

17. The device assembly (10) according to one of claims 13 to 16, **characterized in that** the control unit (14) is attached to the single-use device.

18. The device assembly (10) according to one of claims 13 to 16, **characterized in that** the control unit (14) is located remote from the single-use device (12).
